# FASCICULE DE BREVET EUROPEEN

(11) **EP 2 139 503 B1**
(45) Date de publication et mention de la délivrance du brevet: **08.09.2010**
(21) Numéro de dépôt: 07705468.2
(22) Date de dépôt: 26.01.2007
(51) Int. Cl.: A61K 36/02, A61K 8/73, A61P 17/00, A61K 36/03, A61Q 19/00, A61K 9/00

(54) **COMPOSITIONS COSMETIQUES ET DERMATOLOGIQUES A BASE D'EXTRAITS D'ALGUES**
KOSMETISCHE UND DERMATOLOGISCHE ZUSAMMENSETZUNGEN AUF BASIS VON ALGENEXTRAKTEN
COSMETIC AND DERMATOLOGICAL COMPOSITIONS BASED ON ALGAE EXTRACTS

(30) Priorité: 30.01.2006 FR 0600813
(43) Date de publication de la demande: 06.01.2010
(73) Titulaire: TEXINFINE S.A., 69006 Lyon (FR); Innovat, Parc des Expositions Le Kram, BP N° 1 Tunis (TN); Girmaud, Jean Alexis, 75006 Paris (FR)
(72) Inventeur: GRIMAUD, Jean-Alexis, F-75006 Paris (FR); GUTIERREZ, Gilles, 69450 Saint Cyr au Mont d'or (FR)
(74) Mandataire: Burtin, Jean-François
(86) Numéro de dépôt international: PCT/IB2007/000177
(87) Numéro de publication internationale: WO 2007/085946

(56) Documents cités:
- FR-A- 2 743 723
- FR-A- 2 774 292
- FR-A- 2 803 523
- FR-A- 2 811 563
- FR-A- 2 838 459
- DATABASE WPI Section Ch, Week 200279 Derwent Publications Ltd., London, GB; Class D21, AN 2002-729354 XP002387191 & KR 2002 030 141 A (ENPRANI CO LTD) 24 avril 2002 (2002-04-24)

## Description

La présente invention se rapporte au domaine des nécessités de la vie et plus particulièrement au domaine des compositions destinées à améliorer ou traiter la peau.

L'invention a plus particulièrement pour objet des compositions cosmétiques ou dermatologiques associant 2 extraits d'algues pour accroître d'une manière importante la stabilité des β-endorphines dans les cultures de tissu tout en stimulant très fortement l'expression de β-endorphine et des héparane sulfates par les cellules

L'invention a spécifiquement pour objet des compositions cosmétiques ou dermatologiques caractérisées en ce qu'elles contiennent comme principe actif une association d'extraits de la micro-algue Cyclotelle et de l'algue *Padina pavonica,* dont on connaît l'effet stimulant sur l'expression des protéoglycanes, dans un excipient ou dans un véhicule inerte non-toxique permettant l'application sur la peau.

Une telle préparation possède la propriété d'augmenter sensiblement la production de β-endorphine dans les tissus. La cinétique de dégradation de la β-endorphine montre normalement une dégradation à plus de 50 % au bout de 3 heures. Les effets de l'association selon l'invention résident dans le fait qu'on retarde très notablement la dégradation de la β-endorphine, et que l'on obtient ainsi une stabilité pendant au moins 8 heures, voire même plus de 24 heures.

On connaît déjà l'effet de l'extrait de Cyclotelle sur l'expression de la proopiomélanocortine par les cellules, notamment de la β-endorphine. Cet effet est décrit dans les brevets français n° 2774292 (INOVAT) et n° 2838459 (au nom de COMBE Pierre), le produit utilisé étant un extrait éthanolique ou acétonique. On a constaté que la réponse cellulaire est dose-dépendante et que l'extrait de Cyclotelle reste actif de façon statistiquement significative jusqu'à une dilution de 1/2000. L'induction de l'expression d'héparane sulfate résultant de la présence d'extrait de *Padina pavonica* assure une protection de la β-endorphine, qui est dégradée à une vitesse beaucoup plus lente : 40 à 50 % de cette molécule sont préservés après 24 heures d'incubation d'une culture cellulaire. L'induction de l'expression de l'héparane sulfate endogène constitue donc à la fois un réservoir et une protection, permettant en même temps de stocker les molécules de β-endorphine et de préserver leur intégrité structurale pour une activité biologique plus intense et surtout plus durable.

On connaît depuis les années 1970 le rôle important joué par les endorphines et par les enképhalines dans la perception de la douleur. Cette sécrétion de molécules opioïdes ou peptidiques, d'origine cérébrale ou périphérique, constitue un mécanisme normal de lutte contre la douleur et contribue à la sensation de bien être, de confort ou de plaisir. Cependant en raison du caractère labile de telles molécules leur effet est transitoire et leur utilisation contre la douleur s'en trouve très limitée. Il a donc fallu modifier chimiquement la structure des enképhalines pour arriver à obtenir une molécule stable, résistant aux processus physiologiques de dégradation. Une telle transformation chimique s'accompagne souvent d'une baisse importante du niveau d'activité ou même d'une disparition de l'effet antalgique.

Il était donc important de disposer d'un moyen susceptible d'assurer d'une manière durable l'intégrité de la structure des endorphines et principalement de la β-endorphine d'une manière durable, sans affecter son niveau d'activité. L'induction d'une expression accrue d'héparane sulfate par un extrait d'algues, en particulier par un extrait éthanolique ou acétonique de l'algue *Padina pavonica*, permet de réaliser cet objectif.

L'essence de l'invention réside dans l'obtention d'une expression conjointe d'héparane sulfate. L'expression d'héparane sulfate (protéo glycane) et de β-endorphine est obtenu par des extraits d'algues ; tout extrait de plante susceptible d'induire une surexpression de protéoglycane sulfaté peut être utilisé.

Le terme "héparane sulfate" est un terme général qui sert à désigner d'une manière globale, tout poly saccharide dérivé de l'héparane, naturel, synthétique ou recombinant, d'un poids moléculaire variant de 6.000 à 30.000 Da.

Les compositions selon l'invention comportent en outre des véhicules ou des diluants lipophiles, hydrophiles ou amphiphiles, qui permettent l'application sur la peau d'une quantité efficace de préparation pour assurer une expression importante et durable de β-endorphine.

Le document antérieur FR-A-2743723 (Texinfine) décrit l'utilisation d'algues de la famille des Dictyotales, telles que Padina pavonica, en vue de la réalisation de formules notamment de médicaments destinés à prévenir ou guérir les lésions tissulaires de la peau, de l'os et du cartilage. Les algues de la famille des Dictyotales sont utilisées en vue de leurs actions spécifiques sur la synthèse des glycoaminoglycanes tel que l'acide héparane sulfurique.

Les essais biologiques rapportés ci-après mettent en évidence le niveau élevé d'activité des compositions selon l'invention sur l'expression et la stabilité de la β-endorphine, permettant ainsi d'amener une sensation de soulagement et/ou de plaisir, durable, après un traumatisme ou une plaie importante.

### 1 - ACTIVITE BIOLOGIQUE DE L'EXTRAIT DE CYCLOTELLE

### 1 - Expression de β-endorphine

### But :

Evaluer l'activité biologique d'un extrait brut de Cyclotelle en mesurant l'expression de β-endorphine par les kératinocytes humains (HaCaT) en culture.

### Protocole :

### Traitement des cellules :

Les kératinocytes sont ensemencés à une densité de 50.000 cellules /puits (microplaques 96 puits) dans 200 µl de milieu de culture. Après une nuit d'incubation, les surnageants sont éliminés, puis remplacés par le même volume de milieu neuf seul (contrôle) ou contenant 0.5 % d'une solution éthanolique de l'extrait de Cyclotelle (flacon de 5 ml). Les cellules ainsi traitées sont incubées pendant 48 heures.

A la fin de l'incubation, les surnageants sont éliminés et les tapis cellulaires sont rincés 3 x 5 minutes dans 300 µl de tampon PBS, puis fixés dans un volume de 50 µl d'une solution à 4 % de paraformaldéhyde contenant 0.01 % de Triton X-100. Après 30 minutes de fixation à 4° C. les tapis cellulaires sont rincées plusieurs fois dans le tampon PBS suivi immédiatement du marquage de β-endorphine à l'aide de la technique d'immunofluorescence indirecte.

### Mesure de la fluorescence

A la fin de la réaction d'immunofluorescence, les cellules sont lysées grâce à un tampon de lyse permettant la mise en solution de la fluoresceine. Les surnageants fluorescents ainsi obtenus sont lus au spectrofluorimètre (Fluoroskan) à une longueur d'onde d'excitation de 485 nm et à une longueur d'onde d'émission de 518 nm. Les valeurs obtenues sont des valeurs arbitraires mais proportionnelles à la quantité de β-endorphine marquée.

### Conclusion

Les résultats montrent que l'addition de l'extrait de Cyclotelle dans le milieu de culture stimule très fortement l'expression de β-endorphine par les cellules kératinocytaires. Il est à noter que la réponse cellulaire est dose-dépendante et que l'effet de l'extrait de Cyclotelle reste statistiquement significatif jusqu'à une dilution de 1/2000.

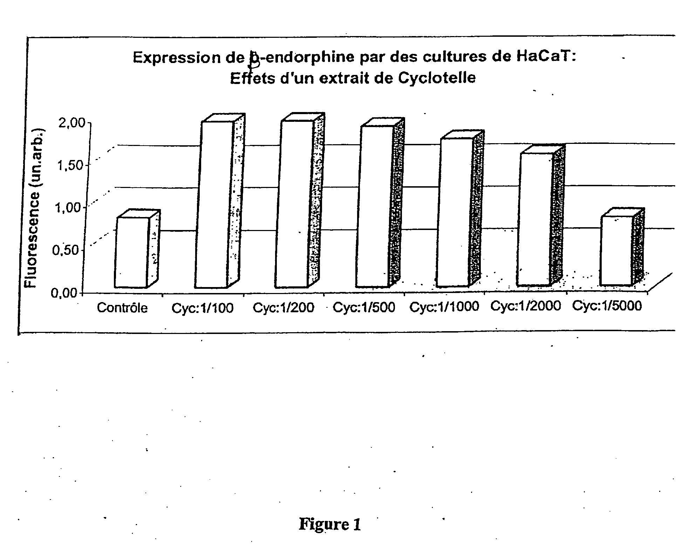

Les résultats obtenus sont représentés à la figure 1 ci-après annexée.

### 2-Stabilité de β-endorphine dans le surnageant de culture.

Les résultats obtenus sur la cinétique de dégradation de la β-endorphine montrent une disparition beaucoup plus rapide du neuropeptide dans les milieux PBS et surnageant de culture, en absence de l'héparane sulfate. Cependant, à partir de 2 heures d'incubation, on observe que la vitesse de dégradation de β-endorphine est plus accélérée dans le surnageant de culture par rapport au tampon PBS, en conséquence de la présence de certains enzymes de dégradation comme les protéases. En revanche, lorsque l'héparane sulfate est présent, aussi bien dans le tampon PBS que dans le surnageant de culture, la β-endorphine est dégradée à une vitesse beaucoup plus lente, préservant ainsi entre 40 et 50 % des molécules après 24 heures d'incubation à température ambiante contre seulement 9 % environ en l'absence de l'héparane sulfate.

La présence de l'héparane sulfate constitue un réservoir qui va permettre à la fois de stocker les molécules de β-endorphine et de préserver leur intégrité structurale pour une activité biologique plus intense et plus durable.

### II - STABILITE DE β-ENDORPHINE ENDOGENE

### But

Mesurer la cinétique de dégradation de β-endorphine néosynthétisée par les kératinocytes dans le surnageant de culture, en présence ou en absence d'héparane sulfate.

### Protocole

La cinétique de dégradation de β-endorphine endogène a été mesurée dans le milieu extracellulaire c'est-à-dire dans le surnageant de culture dans lequel le neuromédiateur a été libéré, par les kératinocytes pour enrichir le surnageant de culture en β-endorphine. Les cellules ont été incubées en présence de l'extrait de Cyclotelle. Après 24 heures d'incubation le milieu conditionné kératinocytaire est récupéré puis divisé en 2 parties dont l'une sera supplémentée avec de l'héparane sulfate à la concentration de 5 µg/ml. Ces 2 milieux contenant de la β-endorphine en présence ou en l'absence d'héparane sulfate sont ensuite incubés à température ambiante (22+/-1° C), puis les concentrations du neuropeptide sont immédiatement déterminées par la technique d'immunofluorescence indirecte après 1. 2, 3. 4. 8 et 24 heures. Les densités de fluorescences sont quantifiées à l'aide d'un spectrofluorimètre (Fluoroscan) à une longueur d'onde d'excitation de 485 nm et une longueur d'onde d'émission de 518 nm.

Le choix d'étudier la cinétique de dégradation de la β-endorphine dans le surnageant de culture a été fait afin de reproduire plus ou moins exactement les propriétés physiologiques du milieu extracellulaire de l'organisme dans lequel le neuromédiateur est naturellement libéré. Ainsi, dans les milieux extra cellulaires "*in vivo*" comme "*in vitro*" se trouvent de nombreuses autres molécules biologiquement actives, notamment des enzymes de dégradation, qui peuvent interférer dans la stabilité et l'activité de la β-endorphine.

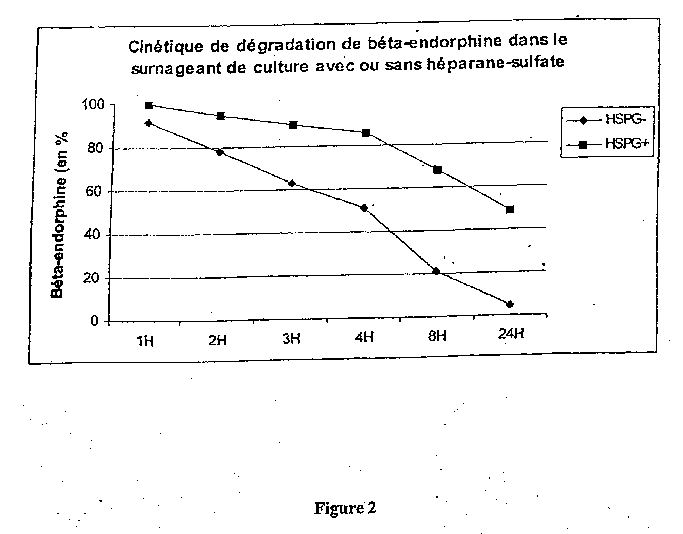

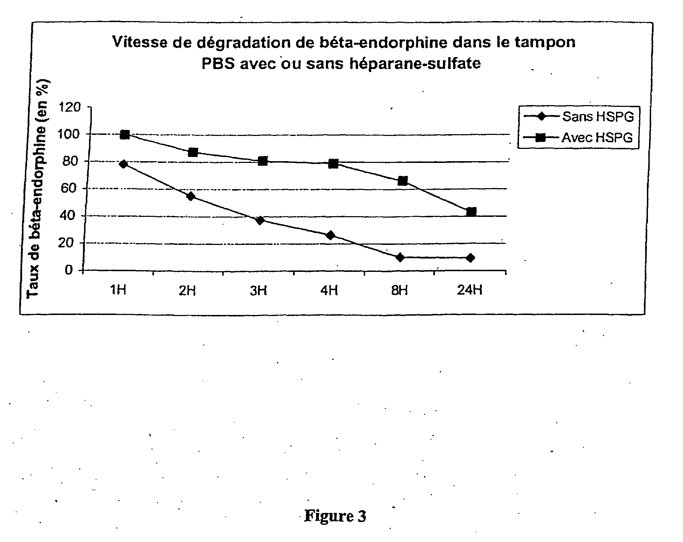

Ces résultats sont mis en évidence dans les figures 2 et 3 ci-après annexées.

### Système neuro-cutané

Le système nerveux et le tissu cutané sont très étroitement liés à la fois par leur origine embryonnaire (le système nerveux et la peau proviennent tous les deux du même tissu, l'ectoderme) et par l'existence de connexions cutanéo-nerveuses distales, les prolongements dendritiques des cellules neuronales étant présentes aussi bien dans le derme que dans l'épiderme. Les cellules nerveuses ont de véritables contacts avec les cellules cutanées et notamment avec les kératinocytes.

### Neuromédiateurs cutanés

On a mis en évidence au niveau de la peau humaine la présence de nombreux neuromédiateurs, parmi lesquels on peut citer la substance P, le neuropeptide Y, le CGRP (calcitonin gene-related peptide), le VIP (vasoactive intestinal peptide) et les POMC (proopiomelanocortines). Ces neuromédiateurs sont libérés par les cellules nerveuses elles-mêmes. Ainsi, les populations kératinocytaires et fibroblastiques sont capables de synthétiser les POMC dans des conditions normales en quantités non négligeables.

### β-endorphine

Il s'agit d'un neuromédiateur et plus exactement d'une neurohormone appartenant à la famille des mélanocortines, qui inclut aussi d'autres molécules comme l'ACTH, MSH et Metenképhaline. Ces neuropeptides proviennent tous de la même protéine qui est la proopiomelanocortine. Cette dernière subit un clivage protéolytique pour se scinder en plusieurs petites molécules peptidiques (citées ci-dessus) biologiquement actives. Toutefois, la β-endorphine reste le représentant principal de cette famille, surtout en raison de son rôle physiologique important dans l'organisme. En effet, la β-endorphine est connue pour ses effets divers, notamment ses effets analgésiques et anti-inflammatoires provoquant des sensations de confort, de bien-être, de plaisir voire d'euphorie. Il est évident que la molécule de β-endorphine présente un grand intérêt, ceci non seulement pour les propriétés évoquées précédemment, mais aussi parce qu'elle peut être synthétisée sur place par des cellules de la peau. L'activité de synthèse de ces cellules peut être modulée, ouvrant ainsi la voie à une cosmétologie active orientée vers l'obtention du confort, du bien-être, et du plaisir.

La sécrétion de β-endorphine peut être stimulée par plusieurs facteurs comme le sport, les variations de température et les pressions mécaniques exercées sur la peau (contact, massage...). Ainsi lorsque son taux augmente suffisamment dans le sang, la β-endorphine procure chez l'individu une sensation graduelle mais fugace de bien-être et de plaisir.

### Stabilité de l'activité biologique de β-endorphine

Comme toute molécule de nature peptidique, la β-endorphine est très instable dans les milieux biologiques. Sa durée de vie est rendue très courte par la présence dans le milieu intercellulaire d'enzymes de dégradation, regroupés sous l'appellation générale de protéases. En effet, aussitôt synthétisées par les cellules et libérées dans le milieu extracellulaire, les molécules de β-endorphine sont aussitôt dégradées par ces protéases. Pour prolonger dans le temps les sensations de bien être et de plaisir, il est donc nécessaire de maintenir stable ce type de peptide.

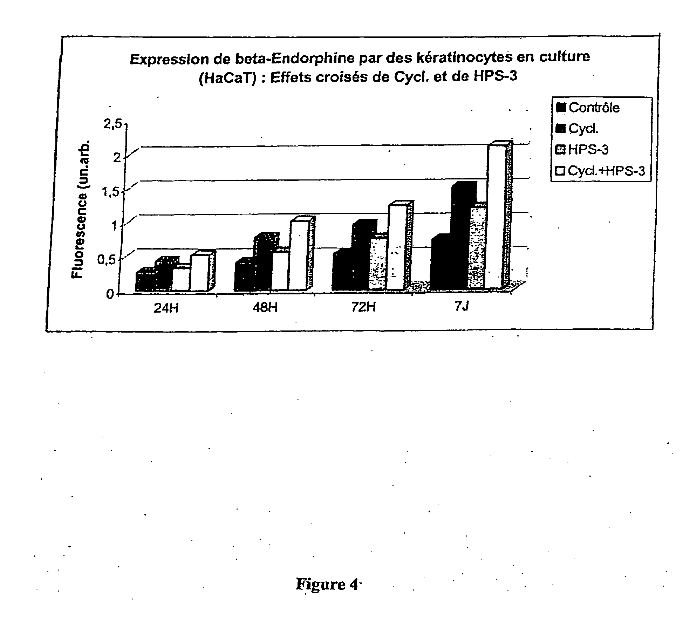

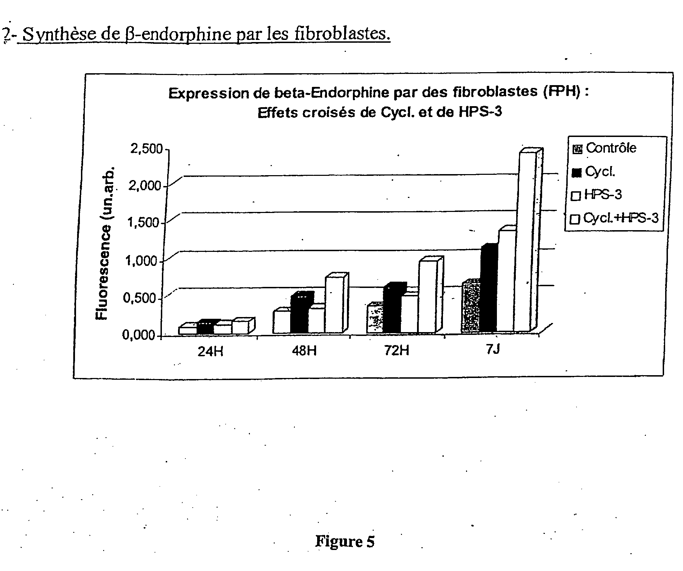

Les résultats obtenus sur l'expression de β-endorphine par les kératinocytes et par les fibroblastes sont représentés aux figures 4 et 5 ci-après annexées.

Les dosages de β-endorphine intra et extracellulaire montrent une stimulation importante de l'expression du neuropeptide par les cultures de kératinocytes et de fibroblastes, après addition de l'extrait de Cyclotelle seul ou associée à un extrait de *Padina pavonica* et ce, dès les premières 24 heures d'incubation. Toutefois, on constate que cette expression est de plus en plus élevée en fonction du temps, dès lors que l'extrait de Cyclotelle est associé à un extrait de

### Padina pavonica.

Par ailleurs, on note une induction significative de l'expression de β-endorphine par un extrait de *Padina pavonica* seul à partir de 48 H pour les kératinocytes et 72 H pour les fibroblastes. Cette induction est liée indirectement à l'effet réservoir de l'héparane sulfate dont la synthèse est largement stimulée par un extrait de *Padina pavonica,* ce qui explique aussi l'efficacité plus importante de l'extrait de Cyclotelle lorsqu'il est associé à un extrait de *Padina pavonica.* En effet, comme ce dernier augmente la synthèse des protéoglycanes et notamment de l'héparane-sulfate, on assiste à une fixation en grand nombre des molécules de β-endorphine par cette matrice extracellulaire riche en protéoglycanes, qui va les accumuler au fil du temps tout en préservant leur activité biologique comme il est montré aux figures 4 et 5.

### Comment faire durer le plaisir ?

L'effet clinique insuffisant observé avec l'extrait de Cyclotelle est dû à la disparition rapide par dégradation extracellulaire, de β-endorphine. On a donc recherché un procédé capable de stabiliser la molécule très rapidement au moment de sa synthèse. Il a été trouvé qu'une quantité importante d'héparane sulfate administrée localement permet de stabiliser la molécule. En effet, ce protéoglycane est connu pour développer une forte affinité vis-à-vis de nombreuses molécules physiologiques. Les molécules liées à l'héparane sulfate deviennent beaucoup plus stables et sont de plus en plus actives. Pour induire la stabilité recherchée il est apparu judicieux de stimuler la synthèse de l'héparane sulfate en rajoutant à l'extrait de Cyclotelle un autre composant comme l'extrait de *Padina pavonica* dont on connaît l'effet stimulant sur l'expression des protéoglycanes, notamment de l'héparane-sulfate.

Par ailleurs, il est à noter que dans le tissu cutané, l'héparane sulfate est localisé surtout au niveau de la membrane basale et est synthétisé aussi bien par les kératinocytes (cellules épidermiques) que par les fibroblastes (cellules dermiques). La présence de ce protéoglycane en quantités suffisantes va donc permettre la fixation et par conséquent la stabilisation d'un plus grand nombre de molécules de β-endorphine, et augmente ainsi leur biodisponibilité. Le rôle réservoir que joue l'héparane sulfate pour les molécules de β-endorphine est d'une importance capitale pour le maintien de leur activité et pour la prolongation de l'effet clinique désiré.

### Synthèse de β-endorphine par les kératinocytes humains

Effets croisés de l'extrait de Cyclotelle et d'extrait de Padine.

### Méthode

On mesure l'effet de l'extrait de Padine qui est un stimulant de l'expression des protéoglycanes, sur la cinétique de synthèse de la β-endorphine par les kératinocytes et les fibroblastes en présence d'extrait de Cyclotelle.

### Protocole

### Traitement des cellules

Les kératinocytes ou les fibroblastes sont ensemencés à une densité de 50.000 cellules par puits (microplaques 96 puits) dans 200 µl de milieu de culture. Après 1 nuit d'incubation, les surnageants sont éliminés, puis remplacés par le même volume de milieu neuf seul (contrôle) ou contenant :
- soit l'extrait de Cyclotelle
- soit l'extrait de Padine
- soit l'extrait de Cyclotelle + un extrait de *Padina pavonica*

Les cellules ainsi traitées sont incubées pendant 24 heures, 48 heures, 72 heures et 7 jours. Les milieux de culture et les traitements sont renouvelés tous les 2 jours.

A la fin de chaque intervalle de temps, les surnageant sont éliminés et les tapis cellulaires sont rincés 3 x 5 dans 300 µl du tampon PBS, puis fixés dans un volume de 50 µl d'une solution à 4 % de paraformaldehyde contenant 0.01 % de Triton X-100. Après 30 minutes de fixation à 4° C, les tapis cellulaires sont rincés plusieurs fois dans le tampon PBS suivi immédiatement du marquage de la β-endorphine à l'aide de la technique d'immunofluorescence indirecte.

### Mesure de la fluorescence

A la fin de la réaction d'immunofluorescence, les cellules sont lysées grâce à un tampon de lyse permettant la mise en solution de la fluorescéine. Les surnageants fluorescents ainsi obtenus sont lus au spectrofluorimètre (Fluroskan) à une longueur d'onde d'excitation de 485 nm et à une longueur d'onde d'émission de 518 nm. Les valeurs obtenues sont des valeurs arbitraires mais proportionnelles à la quantité de β-endorphine marquée.

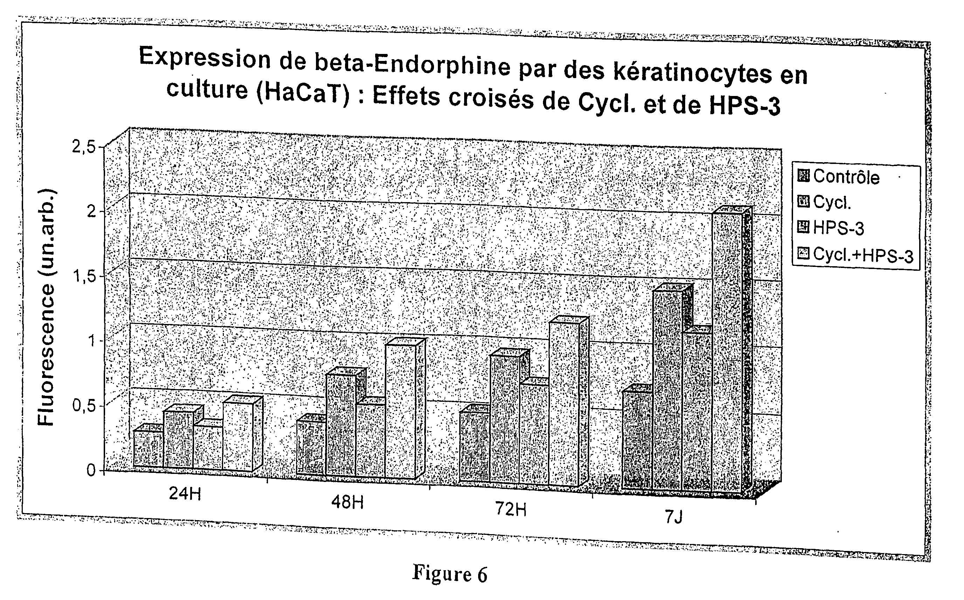

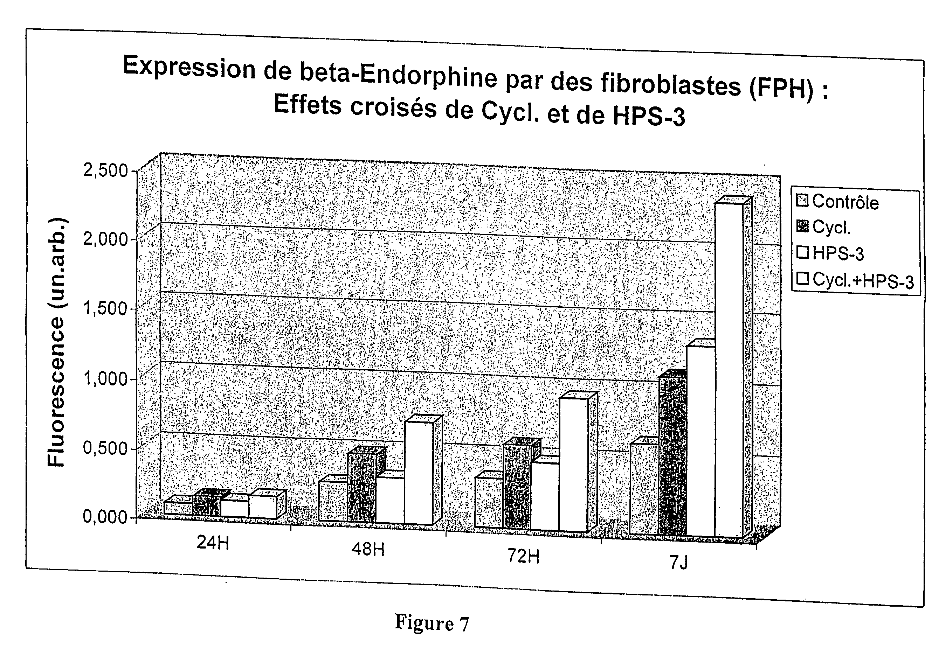

Les valeurs obtenues sont rassemblées dans les tableaux 1 et 2 ci-après et dans les figures 6 et 7 ci-après annexées.

**Tableau 1**

| **1 - Synthèse de β-endorphine par les kératinocytes** | | | | |
|---|---|---|---|---|
| | **24 H** | **48 H** | **72 H** | **7 J** |
| **Contrôle** | 0,274 | 0,412 | 0,529 | 0,74 |
| **Cycl.** | 0,436 | 0,775 | 0,961 | 1,507 |
| **Extrait de Padine** | 0,332 | 0,559 | 0,757 | 1,196 |
| **Cycl.+Extrait de Padine** | 0,524 | 1,014 | 1,239 | 2,102 |

**Tableau 2**

| **2 - Synthèse de β-endorphine par les fibroblastes** | | | | |
|---|---|---|---|---|
| | **24 H** | **48 H** | **72 H** | **7 J** |
| **Contrôle** | 0,081 | 0,282 | 0,355 | 0,649 |
| **Cycl.** | 0,139 | 0,495 | 0.607 | 1.126 |
| **Extrait de Padine** | 0,113 | 0,328 | 0,485 | 1.346 |
| **Cycl.+Extrait de Padine** | 0,158 | 0,736 | 0,952 | 2,385 |

**Tableau 3**

| **Cinétique de dégradation de la β-endorphine** | | | | | | |
|---|---|---|---|---|---|---|
| Qté β-End. | Valeur 1 | Valeur 2 | Valeur 3 | Valeur 4 | Moyenne | Ecart-type |
| 0,25 ng/ml | 0,072 | 0,07 | 0,065 | 0,069 | **0,069** | 0,003 |
| 0,5 ng/ml | 0,135 | 0,127 | 0,119 | 0,121 | **0,126** | 0,007 |
| 1 ng/ml | 0,173 | 0,168 | 0,161 | 0,166 | **0,167** | 0.005 |
| 2 ng/ml | 0,274 | 0,276 | 0,27 | 0,272 | **0,273** | 0,003 |
| 4 ng/ml | 0,402 | 0,406 | 0,3999 | 0,398 | **0,401** | 0,004 |
| 8 ng/ml | 0,73 | 0,734 | 0,686 | 0,62 | **0,693** | 0,053 |
| 16 ng/ml | 1,085 | 1,069 | 1,01 | 1,019 | **1,046** | 0,037 |

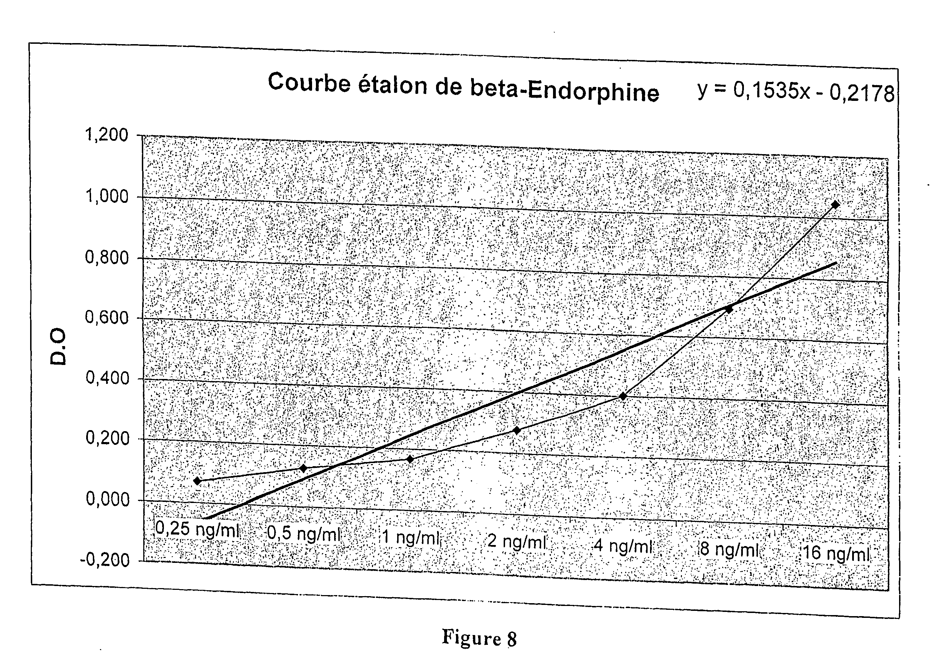

Ces résultats sont figurés graphiquement à la figure 8.

**Tableaux 4 et 5**

| Concentration d'héparane sulfate utilisée : 1 ug/ml (pour saturer les puits). Concentration de β-Endorphine utilisée : 5ng/ml (déterminée d'après la courbe étalon). | | | | | |
|---|---|---|---|---|---|
| Sans H-S | Valeur 1 | Valeur 2 | Valeur 3 | **Moyenne** | Ecart-type |
| 0H | 0,39 | 0,416 | 0,425 | **0,410** | 0,018 |
| 1H | 0,328 | 0,316 | 0,317 | **0,320** | 0,007 |
| 2H | 0,22 | 0,224 | 0,231 | **0,225** | 0,006 |
| 3H | 0,154 | 0,156 | 0,149 | **0,153** | 0,004 |
| 4H | 0,103 | 0,111 | 0,107 | **0,107** | 0,004 |
| 8H | 0,049 | 0,038 | 0,04 | **0,042** | 0,006 |
| 24H | 0,048 | 0,06 | 0,036 | **0,048** | 0,012 |

| Avec H-S | Valeur 1 | Valeur 2 | Valeur 3 | **Moyenne** | Ecart-type |
|---|---|---|---|---|---|
| 0H | 0,359 | 0,401 | 0,419 | **0,393** | 0,031 |
| 1H | 0,427 | 0,388 | 0,385 | **0,400** | 0,023 |
| 2H | 0,341 | 0,341 | 0,347 | **0,343** | 0,003 |
| 3H | 0,321 | 0,311 | 0,321 | **0,318** | 0,006 |
| 4H | 0,294 | 0,308 | 0,327 | **0,310** | 0,017 |
| 8H | 0,273 | 0,248 | 0,259 | **0,260** | 0,013 |
| 24H | 0,161 | 0,17 | 0,177 | **0,169** | 0,008 |

**Tableau 6 et 7**

| **Milieu conditionné des kératinocytes récolté après 24 heures de culture** | | | | | |
|---|---|---|---|---|---|
| M.C sans H-S | Valeur 1 | Valeur 2 | Valeur 3 | **Moyenne** | Ecart-type |
| 0H | 0,478 | 0,517 | 0,525 | **0,507** | 0,025 |
| 1H | 0,413 | 0,429 | 0,461 | **0,434** | 0,024 |
| 2H | 0,271 | 0,241 | 0,261 | **0,258** | 0,015 |
| 3H | 0,183 | 0,166 | 0,153 | **0,167** | 0,015 |
| 4H | | 0,097 | 0,109 | **0,103** | 0,008 |
| 8H | | 0,062 | 0,061 | **0,062** | 0,001 |
| 24H | 0,049 | 0,046 | 0,047 | **0,047** | 0,002 |

| M.C avec H-S | Valeur 1 | Valeur 2 | Valeur 3 | **Moyenne** | Ecartype |
|---|---|---|---|---|---|
| 0H | 0,557 | 0,585 | 0,588 | **0,577** | 0,017 |
| 1H | 0,599 | 0,578 | 0,575 | **0,584** | 0,013 |
| 2H | 0,521 | 0,51 | 0,516 | **0,516** | 0,006 |
| 3H | 0,481 | 0,466 | 0,462 | **0,470** | 0,010 |
| 4H | 0,423 | 0,45 | 0,449 | **0,441** | 0,015 |
| 8H | 0,388 | 0,392 | 0,396 | **0,392** | 0,004 |
| 24H | 0,308 | 0,263 | 0,26 | **0,277** | 0,027 |

Les compositions cosmétiques ou dermatologiques selon l'invention, contiennent de 0,001 mg à 0,1 mg d'extrait de Cyclotelle et de 0,005 mg à 5 mg d'extrait de *Padina pavonica* pour cent g et de préférence de 0,02 mg à 0,5 mg d'extrait de Cyclotelle et de 0,02 mg à 1 mg d'extrait de *Padina pavonica* pour 100 g de préparation.

Les compositions selon l'invention se présentent sous forme de crèmes, de gels, de laits. d'émulsions O/W ou W/O, de liposomes ou de suspensions, de bâtonnets, de sticks, de mousses ou de shampooings ou de lotions.

Les excipients usuels sont des matières grasses, des triglycérides à chaîne moyenne, des alcools gras, des cires ou des phospholipides. Les véhicules sont par exemples des liquides aqueux, des liquides hydro-alcooliques ou des solvants pénétrants. Ils pourront être additionnés d'agents émulsionnants, d'agents épaississants, d'agents tensio-actifs, d'agents parfumants, de colorants ou de gaz propulseur.

Les compositions, selon l'invention sont répartis en flacons, en pots, en tubes, dans des flacons pulvérisateurs, dans des flacons à pompe ou dans des brumisateurs sous pression.

Les compositions selon l'invention trouvent un emploi pour lutter ou faire disparaître les manifestations douloureuses ou d'irritation ou d'inflammations de la peau, des muqueuses ou des téguments, notamment grâce à l'effet prolongé des compositions cosmétiques ou dermatologiques selon l'invention.

## Revendications

1. Compositions cosmétiques et dermatologiques à base d'extrait d'algues, **caractérisées en ce qu'**elles renferment à titre de principe actif une association d'extraits de l'algue Cyclotella et de l'algue *Padina pavonica*, en association ou en mélange avec un excipient ou un véhicule inerte, non toxique, adapté pour l'application topique.

2. Compositions cosmétiques et dermatologiques selon la revendication 1, dans lesquelles l'extrait de Cyclotella est celui obtenu par épuisement éthanolique ou acétonique de l'algue *Cyclotella*.

3. Compositions cosmétiques ou dermatologiques selon la revendication 1, dans lesquelles l'extrait d'algue *Padina pavonica* est un extrait éthanolique ou acétonique de poudre d'algue séchée.

4. Compositions cosmétiques ou dermatologiques selon la revendication 1 et la revendication 2, **caractérisées en ce qu'**elles contiennent de 0,001 mg à 0,1 mg d'extrait de Cyclotelle pour 100g de préparation.

5. Compositions cosmétiques ou dermatologiques selon la revendication 1 et la revendication 2, **caractérisées en ce qu'**elles contiennent de 0,02 mg à 0,5 mg d'extrait de Cyclotelle pour 100 g de préparation.

6. Compositions cosmétiques ou dermatologiques selon la revendication 1 et la revendication 3, **caractérisées en ce qu'**elles contiennent de 0,005 mg à 5 mg d'extrait de *Padina pavonica* pour 100 g de préparation.

7. Compositions cosmétiques ou dermatologiques selon la revendication 6, **caractérisées en ce qu'**elles contiennent de 0,02 mg à 1 mg d'extrait de *Padina pavonica* pour 100 g de préparation

8. Compositions cosmétiques ou dermatologiques selon l'une des revendications précédentes, **caractérisées en ce qu'**elles se présentent sous forme de crèmes, de gels, de laits, de suspensions, d'émulsions O/W ou W/O, de liposomes ou de mousses.

9. Compositions cosmétiques selon la revendication 1, pour l'utilisation dans la suppression des phénomènes d'irritation de la peau, des muqueuses ou des téguments.

10. Utilisation des compositions dermatologiques selon la revendication 1 en vue de la production d'un médicament assurant le soulagement ou la suppression des manifestations douloureuses de la peau, des muqueuses ou des téguments.

11. Utilisation des compositions dermatologiques selon la revendication 10 en vue de la production d'un médicament assurant un effet antalgique prolongé supérieur à 24 heures

## Claims

1. Cosmetic and dermatological compositions based on an extract of algae, wherein they contain, as the active ingredient, a mixture of extracts of the alga Cyclotella and of the alga Padina pavonica in admixture or combination with an inert non toxic carrier or vehicle, suitable for the topic application.

2. Cosmetic and dermatological compositions according to claim 1 wherein the extract of Cyclotella is that obtained through ethanolic or acetonic extraction of the powder of the alga Cyclotella.

3. Cosmetic or dermatological compositions according to claim 1 wherein the extract of alga Padina pavonica is an ethanolic or acetonic extract of the dried alga powder.

4. Cosmetic or dermatological compositions according to any of the preceding claims, wherein they contain 0,001 to 0,1 mg of Cyclotella extract for 100g of preparation.

5. Cosmetic and dermatological compositions according to any of the preceding claims, wherein they contain 0,02 to 0,5 mg of Cyclotella extract for 100g of preparation.

6. Cosmetic or dermatological compositions according to any of the preceding claims, wherein they contain from 0,005 mg to 5 mg of extract of Padina pavonica for 100 g of preparation.

7. Cosmetic or dermatological compositions according to any of the preceding claims, wherein they contain from 0,02 mg to 1 mg of extract of Padina pavonica for 100 g of preparation.

8. Cosmetic or dermatological compositions according to any of the preceding claims wherein they are dispersed under the form of creams, gels, milks, liposomes, or foams.

9. Use of the cosmetic compositions according to claim 1 for the disappearance of the phenomena of irritation of the skin, of the mucous membranes, or of the teguments.

10. Use of the dermatological compositions according to claim 1 for the production of a drug allowing the alleviation or the elimination of the painful symptoms, based on an extract of alga Cyclotella and an extract of alga Padina pavonica.

11. Use of the dermatological compositions according to claim 1 for the production of a drug ensuring a protracted analgesic effect with a duration of more than 24 hours, and based on an extract of alga Cyclotella and an extract of alga Padina pavonica.

## Patentansprüche

1. kosmetische und dermatologische Zusammensetzungen mit dem Grundlage einen Extrakt von Algen, **dadurch gekennzeichnet dass** sie eine Vereinigung von Extrakten der Alge Cyclotella und der Alge Padina als Wirkstoff enthalten, zusammen oder in Bemischung mit einem inerten unschädlichen für die topische Applikation geeigneten Träger oder Vehikel.

2. kosmetische und dermatologische Zusammensetzungen gemäß Anspruch 1, worin der Extrakt von Cyclotella mittels einer ethanolischen oder azetonischen Extraktion der Alge Cyclotella hergestellt wird.

3. kosmetische oder dermatologische Zusammensetzungen gemäß Anspruch 1, worin der Extrakt von Padina pavonica ein ethanolischer oder azetonischer Extrakt des getrockenen Alge Pulver ist.

4. kosmetische oder dermatologische Zusammensetzungen gemäß Anspruch 1 und Anspruch 2, **dadurch gekennzeichnet dass** sie von 0.001 mg bis 0.1 mg Cyclotella Extrakt pro 100g Zubereitung enthalten.

5. kosmetische oder dermatologische Zusammensetzungen gemäß Anspruch 1 und Anspruch 2, **dadurch gekennzeichnet dass** sie von 0.02 mg bis 0.5 mg Cyclotella Extrakt pro 100g Zubereitung enthalten.

6. kosmetische oder dermatologische Zusammensetzungen gemäß Anspruch 1 und Anspruch 2, **dadurch gekennzeichnet dass** sie von 0.005 mg bis 5 mg Padina pavonica Extrakt pro 100g Zubereitung enthalten.

7. kosmetische oder dermatologische Zusammensetzungen gemäß Anspruch 6, **dadurch gekennzeichnet dass** sie von 0.02 mg bis 1 mg Padina pavonica Extrakt pro 100g Zubereitung enthalten.

8. kosmetische oder dermatologische Zusammensetzungen gemäß einem der vorherigen Ansprüchen, **dadurch gekennzeichnet dass** sie unter der Form von Salben, Gellen, Milchen, Suspensionen, o/w oder w/o Emulsionen, Liposomen, oder Schaumen sich vorliegen.

9. Verwendung der kosmetischen Zusammensetzungen gemäß Anspruch 1, die für die Abschaffung der Phänomens der Reizungen des Hauts und der Schleimhäute und der Hülle geeignet sind.

10. Verwendung der kosmetischen Zusammensetzungen gemäß Anspruch 1 für die Herstellung eines Arzneimittels, das die Minderung oder Abschaffung der schmerzhaften Äußerungen versichert, und das aus die Alge Cyclotella und die Alge Padina besteht.

11. Verwendung der kosmetischen Zusammensetzungen gemäß Anspruch 1 für die Herstellung eines Arzneimittels, das eine langdauerliche (mehr als 24 Stünden) antalgische Wirkung versichert, und das aus einem Extrakt von Cyclotella Alge und einem Extrakt von Padina pavonica besteht.
